(19) Europäisches Patentamt
European Patent Office
Office européen des brevets

(11) EP 0 727 410 A1

(12) EUROPÄISCHE PATENTANMELDUNG

(43) Veröffentlichungstag:
21.08.1996 Patentblatt 1996/34

(51) Int Cl.$^{6}$: **C07C 69/753**, C07D 307/93, C08K 5/134

(21) Anmeldenummer: 96810063.6

(22) Anmeldetag: 30.01.1996

(84) Benannte Vertragsstaaten:
BE DE ES FR GB IT NL

(30) Priorität: 08.02.1995 CH 368/95

(71) Anmelder: CIBA-GEIGY AG
4002 Basel (CH)

(72) Erfinder:
- Pitteloud, Rita, Dr.
CH-1724 Praroman (CH)
- Gilg, Bernard, Dr.
F-68300 St. Louis-la-Chaussée (FR)

(54) **Bisphenolesterderivate**

(57) Die neuen Verbindungen der Formel I,

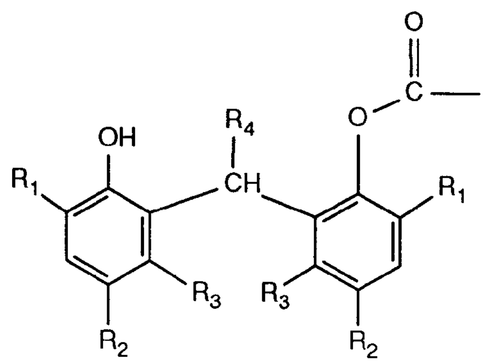

(I)

worin A eine Gruppe der Formel IIa oder IIb bedeutet,

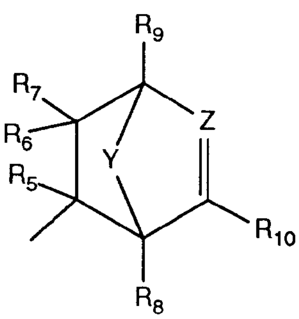

(IIa),

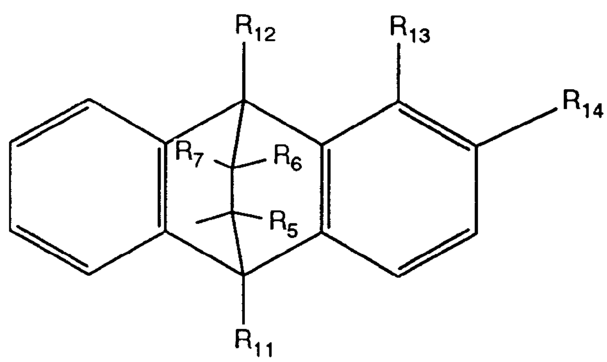

(IIb)

Y Sauerstoff, Methylen, Ethyliden oder eine Gruppe >C=C($CH_3$)$_2$ ist, Z Stickstoff,

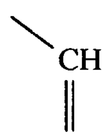

oder

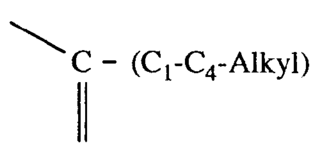

darstellt, $R_1$ und $R_2$ z.B. $C_1$-$C_5$-Alkyl sind, $R_3$ und $R_4$ z.B. Wasserstoff oder Methyl bedeuten und $R_5$, $R_6$, $R_7$, $R_8$, $R_9$, $R_{10}$, $R_{11}$, $R_{12}$, $R_{13}$ und $R_{14}$ z.B. Wasserstoff darstellen, eignen sich zum Stabilisieren von organischem Material gegen oxidativen, thermischen oder lichtinduzierten Abbau.

EP 0 727 410 A1

## Beschreibung

Die vorliegende Erfindung betrifft neue Bisphenolesterderivate, ihre Verwendung und das mit ihrer Hilfe gegen oxidativen, thermischen und lichtinduzierten Abbau stabilisierte organische Material.

Die Verwendung von einigen Bisphenolesterderivaten als Stabilisatoren wird zum Beispiel in JP-A-Hei 4-308 581, DE-A-3 718 751, EP-A-479 560 und US-A-4 414 408 beschrieben.

Ein Gegenstand der vorliegenden Erfindung ist eine Verbindung der Formel I,

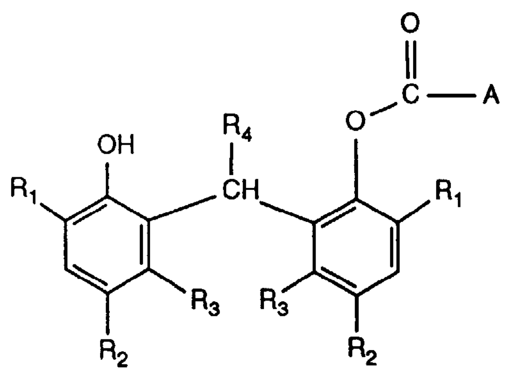

(I)

worin A eine Gruppe der Formel IIa oder IIb bedeutet,

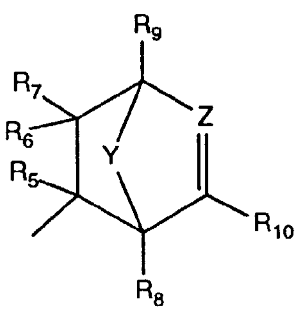

(IIa),

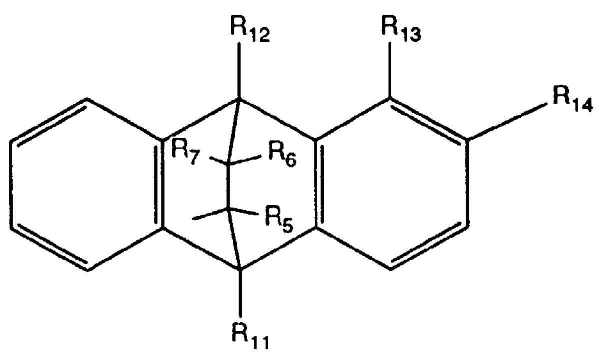

(IIb)

Y Sauerstoff, Methylen, Ethyliden oder eine Gruppe >C=C$(CH_3)_2$ ist,

Z Stickstoff,

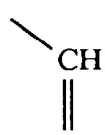

oder

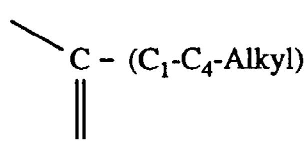

darstellt,

die Reste $R_1$ unabhängig voneinander $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder -$CH_2$-S-$X_1$ bedeuten,

die Reste $R_2$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-

$C_4$-Alkyl substituiertes $C_5$-$C_{12}$-Cycloalkyl, $C_5$-$C_{12}$-Cycloalkenyl, durch $C_1$-$C_4$- substituiertes $C_5$-$C_{12}$-Cycloalkenyl, Phenyl, durch $C_1$-$C_4$-substituiertes Phenyl, $C_7$-$C_9$-Phenylalkyl, $-CH_2-S-X_1$, $-(CH_2)_pCOO-X_2$ oder $-(CH_2)_qO-X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alkyl, Phenyl, $-CH_2-COO-X_4$ oder CN bedeutet,

$R_6$ Wasserstoff, $C_1$-$C_4$-Alkyl, Phenyl, $-COO-X_5$, -CN oder $-CON(X_6)(X_7)$ ist,

$R_7$ Wasserstoff oder $C_1$-$C_{10}$-Alkyl bedeutet,

$R_8$, $R_9$ und $R_{10}$ unabhängig voneinander Wasserstoff oder $C_1$-$C_4$-Alkyl sind,

$R_{11}$ und $R_{12}$ unabhängig voneinander Wasserstoff, $C_1$-$C_4$Alkyl oder Phenyl bedeuten,

$R_{13}$ und $R_{14}$ unabhängig voneinander Wasserstoff oder $C_1$-$C_4$-Alkyl sind,

$X_1$ $C_1$-$C_{25}$-Alkyl, $C_5$-$C_{12}$-Cycloalkyl durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder $-(CH_2)_rCOO-Y_1$ darstellt,

$X_2$, $X_4$ und $X_5$ unabhängig voneinander $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

$X_3$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$X_6$ und $X_7$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl oder $C_7$-$C_9$-Phenylalkyl sind,

$Y_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet.

Alkyl mit bis zu 25 Kohlenstoffatomen, bevorzugt mit bis zu 18 Kohlenstoffatomen, insbesondere mit bis zu 10 Kohlenstoffatomen, bedeutet beispielsweise Methyl, Ethyl, Propyl, Isopropyl, n-Butyl, sec-Butyl, Isobutyl, tert-Butyl, 2-Ethylbutyl, n-Pentyl, tert-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl.

Eine der bevorzugten Bedeutungen von $R_1$ und $R_2$ ist verzweigtes Alkyl mit bis zu 10 Kohlenstoffatomen, insbesondere mit bis zu 5 Kohlenstoffatomen, z.B. tert-Butyl und tert-Pentyl.

Eine der bevorzugten Bedeutungen von $R_4$ ist $C_1$-$C_4$-Alkyl, insbesondere Methyl.

Eine der bevorzugten Bedeutungen von $Y_2$ is $C_1$-$C_4$-Alkyl, insbesondere Methyl.

Alkenyl mit bis zu 24 Kohlenstoffatomen, insbesondere mit bis zu 18 Kohlenstoffatomen, bedeutet beispielsweise Vinyl, Propenyl, Isopropenyl, 2-Butenyl, 3-Butenyl, Isobutenyl, 3-Methyl-but-2-enyl, n-Oct-2-enyl, n-Dodec-2-enyl, iso-Dodecenyl, n-Octadec-2-enyl oder n-Octadec-4-enyl. Alkenylreste, in denen das Kohlenstoffatom in der Stellung 1

gesättigt ist, sind bevorzugt. $C_3$-$C_{18}$-Alkenyl ist besonders bevorzugt.

Unsubstituiertes oder durch $C_1$-$C_4$-Alkyl substituiertes $C_5$-$C_{12}$-Cycloalkyl bedeutet zum Beispiel Cyclopentyl, Cyclohexyl, Cycloheptyl, Cyclooctyl, Cyclododecyl, 2- oder 4-Methylcyclohexyl, Dimethylcyclohexyl, Trimethylcyclohexyl oder tert-Butylcyclohexyl. Unsubstituiertes oder durch $C_1$-$C_4$-Alkyl substituiertes $C_5$-$C_8$-Cycloalkyl, insbesondere Cyclohexyl, ist bevorzugt.

Beispiele für unsubstituiertes oder durch $C_1$-$C_4$-Alkyl substituiertes $C_5$-$C_{12}$-Cycloalkenyl, bevorzugt $C_5$-$C_8$-Cycloalkenyl, sind Cyclohex-2-enyl, Cyclohept-3-enyl und 4-tert-Butylcyclohex-2-enyl. Bevorzugt ist Cyclohexenyl.

Durch $C_1$-$C_4$-Alkyl substituiertes Phenyl ist beispielsweise Methylphenyl, Dimethylphenyl, Trimethylphenyl, Ethylphenyl, Diethylphenyl, Isopropylphenyl, tert-Butylphenyl, Di-tert-butylphenyl oder Methyl-di-t-butylphenyl.

Beispiele für $C_7$-$C_9$-Phenylalkyl, welches gegebenenfalls am Phenylring durch $C_1$-$C_4$-Alkyl substituiert ist, sind Benzyl, Phenethyl, 3-Phenylpropyl, $\alpha$-Methylbenzyl, $\alpha,\alpha$-Dimethylbenzyl, Methylbenzyl, Dimethylbenzyl, Trimethylbenzyl und tert-Butylbenzyl.

Alkanoyl mit bis zu 25 Kohlenstoffatomen bedeutet beispielsweise Methanoyl, Ethanoyl, Propanoyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, octadecanoyl, Nonadecanoyl oder Eicosanoyl. $C_1$-$C_{18}$-Alkanoyl ist eine bevorzugte Bedeutung.

Beispiele für durch Sauerstoff, Schwefel oder >N-$Y_2$ unterbrochenes $C_3$-$C_{25}$-Alkanoyl sind -CO-$CH_2CH_2$-S-($C_1$-$C_{10}$-Alkyl), -CO-$CH_2CH_2$-O-($C_1$-$C_{10}$-Alkyl) und -CO-$CH_2CH_2$-N($Y_2$)-($C_1$-$C_{10}$-Alkyl). Durch Sauerstoff oder >N-$Y_2$ unterbrochenes $C_3$-$C_{25}$-Alkanoyl ist bevorzugt.

Beispiele für $C_3$-$C_{25}$-Alkenoyl sind Acryloyl, Methacryloyl, Crotonoyl, Isocrotonoyl und Oleolyl. $C_3$-$C_{18}$-Alkenoyl ist bevorzugt.

$C_6$-$C_9$-Cycloalkylcarbonyl bedeutet beispielsweise Cyclopentylcarbonyl, Cyclohexylcarbonyl, Cycloheptylcarbonyl oder Cyclooctylcarbonyl.

Durch $C_1$-$C_4$-Alkyl substituiertes Benzoyl bedeutet zum Beispiel Methylbenzoyl oder tert-Butylbenzoyl.

Bevorzugt ist eine Verbindung der Formel I, worin

die Reste $R_1$ unabhängig voneinander $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder -$CH_2$-S-$X_1$ bedeuten,

die Reste $R_2$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, -$CH_2$-S-$X_1$, -$(CH_2)_p$COO-$X_2$ oder -$(CH_2)_q$O-$X_3$ sind,

$R_5$ Wasserstoff, $C_1$-$C_{10}$-Alkyl, Phenyl oder CN bedeutet,

$R_6$ Wasserstoff, $C_1$-$C_4$-Alkyl oder Phenyl ist,

$X_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder -$(CH_2)_r$COO-$Y_1$ darstellt,

$X_2$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl bedeutet,

$X_3$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder >N-$Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$Y_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist.

Ebenfalls bevorzugt ist eine Verbindung der Formel I, worin

die Reste $R_1$ unabhängig voneinander $C_1$-$C_{10}$-Alkyl, $C_2$-$C_{18}$-Alkenyl, $C_5$-$C_6$-Cycloalkyl, $C_5$-$C_8$-Cycloalkenyl, Phenyl, $C_7$-$C_9$-Phenylalkyl oder -$CH_2$-S-$X_1$ bedeuten,

die Reste $R_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, $-CH_2$-S-$X_1$, $-(CH_2)_pCOO$-$X_2$ oder $-(CH_2)_qO$-$X_3$ sind,

die Reste $R_3$ Wasserstoff darstellen,

$R_4$ Wasserstoff oder $C_1$-$C_4$-Alkyl ist,

$R_5$ Wasserstoff, $C_1$-$C_4$-Alkyl, Phenyl oder CN bedeutet,

$R_6$ Wasserstoff, $C_1$-$C_4$-Alkyl oder Phenyl ist,

$R_7$ Wasserstoff oder $C_1$-$C_4$-Alkyl bedeutet,

$X_1$ $C_1$-$C_{10}$-Alkyl, $C_5$-$C_8$-Cycloalkyl, Phenyl, $C_7$-$C_9$-Phenylalkyl oder $-(CH_2)_rCOO$-$Y_1$ darstellt,

$X_2$ $C_1$-$C_{10}$-Alkyl, $C_5$-$C_8$-Cycloalkyl, Phenyl oder $C_7$-$C_9$-Phenylalkyl bedeutet,

$X_3$ $C_1$-$C_{10}$-Alkyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl oder Benzoyl ist,

$Y_1$ $C_1$-$C_{10}$-Alkyl, $C_5$-$C_8$-Cycloalkyl, Phenyl oder $C_7$-$C_9$-Phenylalkyl ist.

Die Reste $R_8$, $R_9$, $R_{10}$, $R_{11}$, $R_{12}$, $R_{13}$ und $R_{14}$ bedeuten bevorzugt Wasserstoff.
Von Interesse ist auch eine Verbindung der Formel I, worin

die Reste $R_1$ unabhängig voneinander $C_1$-$C_5$-Alkyl oder $C_5$-$C_8$-Cycloalkyl bedeuten,

die Reste $R_2$ unabhängig voneinander $C_1$-$C_5$-Alkyl darstellen,

die Reste $R_3$ Wasserstoff bedeuten und

$R_4$ Wasserstoff oder $C_1$-$C_4$-Alkyl ist.

Ebenfalls von Interesse ist eine Verbindung der Formel I, worin

die Reste $R_1$ gleich sind und verzweigtes $C_3$-$C_5$-Alkyl bedeuten,

die Reste $R_2$ gleich sind und $C_1$-$C_5$-Alkyl darstellen,

die Reste $R_3$ Wasserstoff bedeuten und

$R_4$ Wasserstoff oder $C_1$-$C_4$-Alkyl ist.

Besonders bevorzugt ist eine Verbindung der Formel I, worin

Y Sauerstoff oder Methylen bedeutet,

Z eine Gruppe

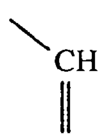

ist,

die Reste $R_1$ gleich sind und verzweigtes $C_3$-$C_5$-Alkyl bedeuten,

die Reste $R_2$ gleich sind und $C_1$-$C_5$-Alkyl darstellen,

die Reste $R_3$ Wasserstoff bedeuten,

$R_4$ Wasserstoff oder $C_1$-$C_4$-Alkyl ist,

$R_5$ Wasserstoff, $C_1$-$C_4$-Alkyl oder CN darstellt,

$R_6$, $R_7$, $R_8$, $R_9$, $R_{10}$, $R_{11}$, $R_{12}$, $R_{13}$ und $R_{14}$ Wasserstoff sind.

Von besonderem Interesse ist die Verbindung der Formel

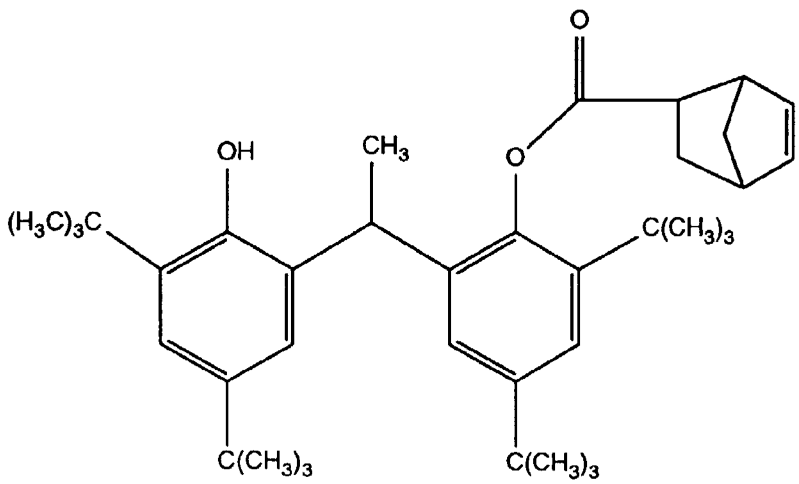
.

Z bedeutet bevorzugt eine Gruppe

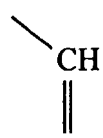
.

Y ist bevorzugt Sauerstoff oder Methylen.

Die Herstellung der Verbindungen der Formel I kann beispielsweise nach folgenden, an sich bekannten Verfahren durchgeführt werden:

Verfahren A: Umsetzung einer Verbindung der Formel a

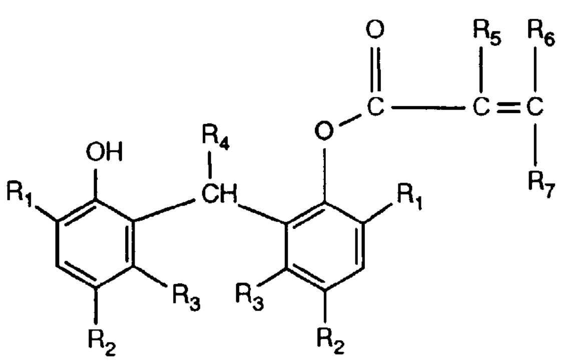
(a)

mit einer geeigneten Verbindung der Formel b-1 oder c (Diels-Alder Reaktion wie z.B. in "Comprehensive Organic

Synthesis, Vol. IV, 315 (1991), Pergamon Press oder Chem. Rev. 1993, 93, 741-761" beschrieben),

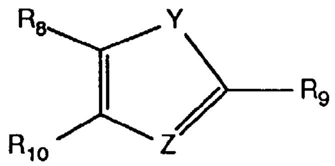 (b-1), 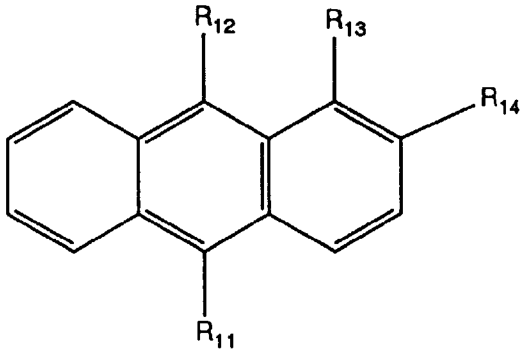 (c)

worin die variablen Reste die oben angegebenen Bedeutungen besitzen.

Diese thermische Diels-Alder Reaktion kann zum Beispiel durch Mischen der beiden Reaktionspartner in einem geeigneten Lösungsmittel bei einer Temperatur zwischen Raumtemperatur und dem Siedepunkt der Reaktionsmischung erfolgen. Bevorzugt wird dabei unter Ausschluss von Sauerstoff gearbeitet Mögliche Lösungsmittel sind übliche Kohlenwasserstoffe (z.B. Toluol, Hexan oder Cyclohexan), halogenierte Kohlenwasserstoffe (z.B. Dichlormethan, Dichlorethan oder Chlorbenzol), Ether (z.B. Diethylether, Tetrahydrofuran oder Dimethoxyethan), Alkohole (z.B. Methanol, Ethanol, Isopropanol), Wasser oder Wasser-Alkohol sowie Wasser-Ether Gemische.

Wenn Y Methylen und Z eine Gruppe

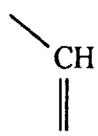

oder

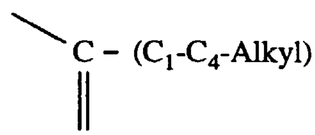

bedeuten, kann an Stelle der Verbindung der Formel b-1 auch ein Dimeres der Formel b-2

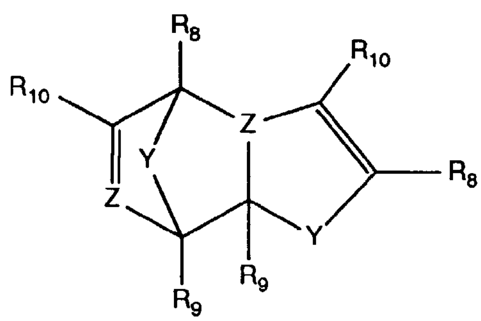 (b-2)

eingesetzt werden. In diesem Fall ist es zweckmässig, die Reaktion bei hohen Temperaturen, z.B. 180-220°C (Krakkungstemperatur eines Dimeren wie Dicyclopentadien), in einem geschlossenen System (Autoklav) unter Druck (z.B. 1-5 bar) durchzuführen. Die Umsetzung kann auch ohne Lösungsmittel erfolgen.

Ferner kann die Reaktion auch bei tiefen Temperaturen (z.B. 0-50°C) in Gegenwart einer Lewissäure als Katalysator durchgeführt werden. Der Katalysator liegt vorzugsweise in Mengen von 2-50 Mol%, insbesondere 2-10 Mol%, vor. Geeignete Lewissäuren sind zum Beispiel R-Al-$Cl_2$ oder $(R)_2$-AlCl, wobei R Methyl, Ethyl, n-Propyl oder Isopropyl bedeutet, oder ferner $AlCl_3$, $SnCl_4$, $ZnCl_2$, $ZnJ_2$, $FeCl_3$ oder $TiCl_4$.

<u>Verfahren B:</u> Umesterung von Carbonsäurederivaten der Formel d oder e,

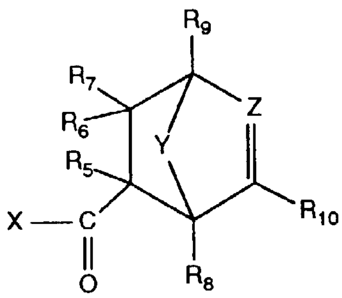 (d), 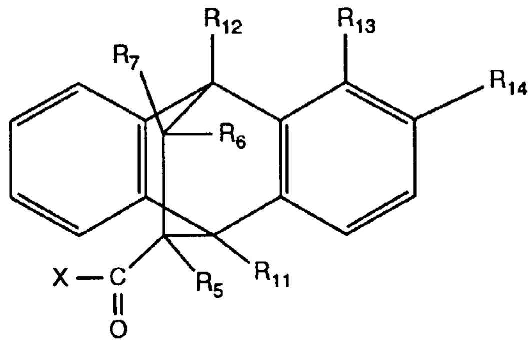 (e)

worin X zum Beispiel OH, Cl,

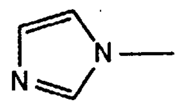

u.s.w. bedeutet, mit einem Bisphenol der Formel f,

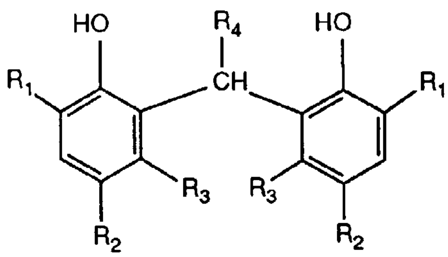 (f)

worin die variablen Reste die oben angegebenen Definitionen haben.

Die Reaktion kann in an sich bekannter Weise durchgeführt werden; zum Beispiel durch Zufügen eines der beiden Edukte zu dem zweiten Edukt und Durchmischen der beiden Reaktionspartner, bevorzugt unter Ausschluss von Sauerstoff. Die Umsetzung kann mit oder ohne Lösungsmittel (z.B. Toluol) durchgeführt werden. Die Temperatur kann z. B. zwischen dem Schmelzpunkt und dem Siedepunkt der Reaktionsmischung liegen, beispielsweise zwischen -50 und 150°C, vorzugsweise zwischen 0 und 150°C. Die Reinigung des erhaltenen Produktes kann ebenfalls nach bekannten Methoden erfolgen, beispielsweise durch Eindampfen des Lösungsmittels und Kristallisation des Rohproduktes oder durch Waschen mit Wasser/HCl, Extraktion mit einem organischen Lösungsmittel, Kristallisation und/oder Chromatographie. Als Lösungsmittel für die Extraktion sowie für den chromatographischen Reinigungsschritt werden Hexan, Essigester oder deren Gemische bevorzugt.

Wird in der Reaktion als Derivat der Carbonsäure der Formel d oder e ein Säurechlorid (X = Cl) eingesetzt, so kann der Reaktionsmischung auch ein Säureakzeptor hinzugefügt werden. Geeignete Säureakzeptoren sind beispielsweise Amine wie Pyridin oder Triethylamin. Bevorzugt ist die Menge des Säureakzeptors mindestens äquivalent der Menge des Säurechlorids. Sie beträgt beispielsweise 1 bis 2 Äquivalente, insbesondere 1,2 bis 1,7 Äquivalente, bezogen auf das Säurechlorid.

Das Säurechlorid kann auch "in situ" hergestellt werden. In diesem Fall werden die Carbonsäure der Formel d oder e (X = OH), das Bisphenol der Formel f und ein Säureakzeptor (z.B. Triethylamin) vorgelegt und anschliessend Phosphoroxychlorid in Analogie zu dem beispielsweise in US-A-5 128 398 beschriebenen Verfahren zugegeben.

Weiter ist es auch möglich, zuerst aus dem Bisphenol der Formel f das entsprechende mono-Natrium, Kalium- oder Lithium-phenolat herzustellen und dann dieses mit dem Säurechlorid umzusetzen. Zur Herstellung der benötigten Phenolate werden Hydride, Alkalimetalle, Alkalihydroxide, Alkalialkoholate oder Alkyllithiumverbindungen als Basen verwendet.

Werden die Carbonsäuren der Formel d oder e (X= OH) und das Bisphenol der Formel f direkt als Edukte eingesetzt, wird die Umsetzung zweckmässigerweise unter Verwendung von Reagenzien durchgeführt, die freigesetztes Wasser aufnehmen, wie z.B. Dicyclohexylcarbodiimid.

Die in den oben beschriebenen Verfahren benutzten Ausgangsstoffe sind bekannt, teilweise im Handel erhältlich

oder können in Analogie zu bekannten Verfahren hergestellt werden.

Die Verbindungen der Formel I fallen als Isomerengemische (endo-, exo-, cis-, trans-Form u.s.w.), die je nach Herstellungsverfahren variieren, an.

Beispiele für Verbindungen der Formel I sind in der folgenden Tabelle 1 aufgeführt.

Tabelle 1:

| Verbindung | Struktur | Elementaranalyse Schmelzpunkt |
|---|---|---|
| 101 | 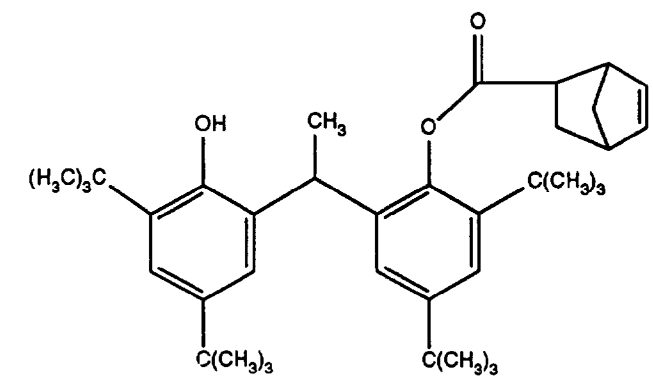 | Elementaranalysen und Schmelzpunkte sind in den Beispielen 1 bis 5 angegeben. |
| 102 | 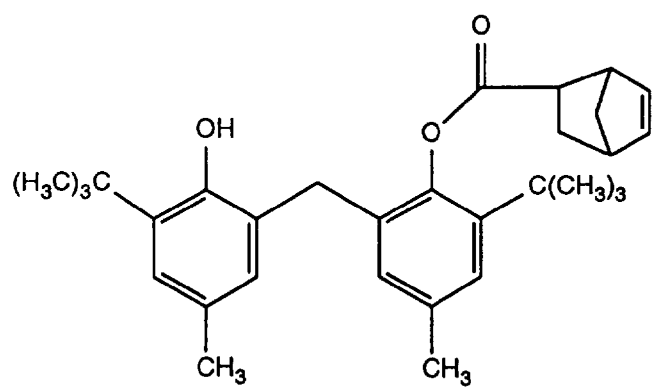 | **Elementaranalyse:**<br>C% H%<br>ber.: 80,83 8,75<br>gef.: 80,81 8,91<br>**Schmelzpunkt:** 107-112°C |

Tabelle 1 (Fortsetzung):

| Verbindung | Struktur | Elementaranalyse Schmelzpunkt |
|---|---|---|
| 102 bis | 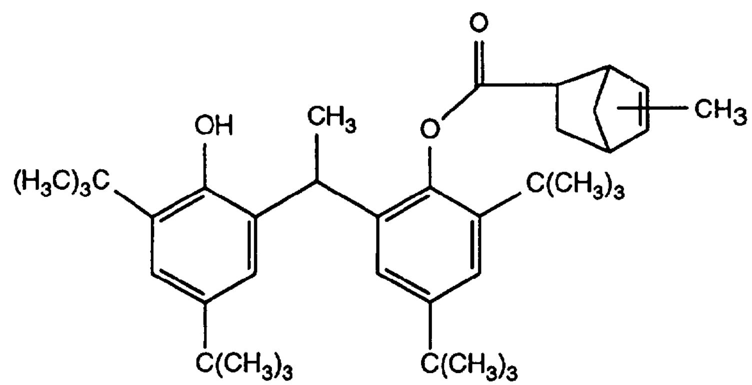 | **Elementaranalyse:**<br>C% H%<br>ber.: 81,77 9,85<br>gef.: 81,71 9,94<br><br>**Schmelzpunkt:** 73-81°C |

Tabelle 1 (Fortsetzung):

| Verbindung | Struktur | Elementaranalyse Schmelzpunkt |
|---|---|---|
| 103 | 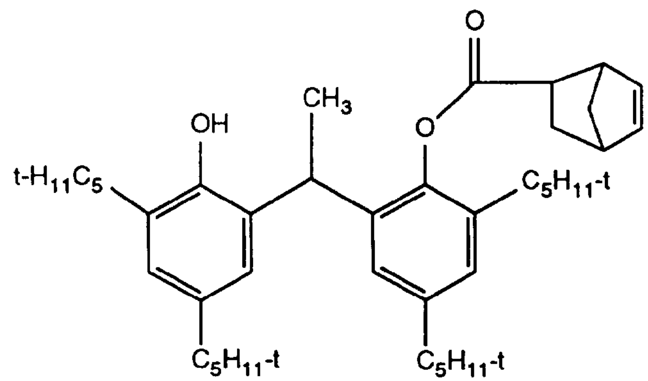 | Elementaranalyse:<br>C% H%<br>ber.: 82,03 10,16<br>gef.: 82,17 10,23<br>Schmelzpunkt: 118-121°C |
| 104 | 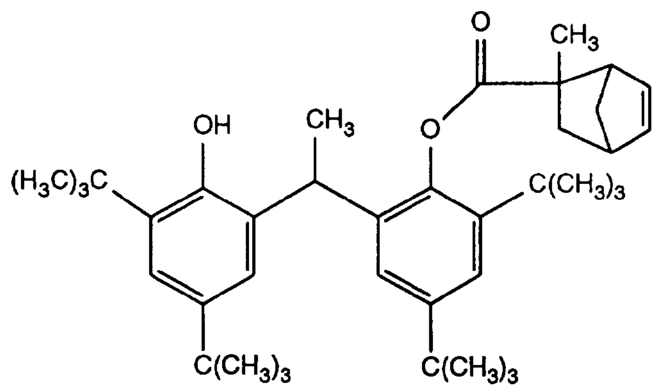 | Elementaranalyse:<br>C% H%<br>ber.: 81,77 9,85<br>gef.: 81,20 9,99<br>Schmelzpunkt: 132-140°C |

Tabelle 1 (Fortsetzung):

| Verbindung | Struktur | Elementaranalyse Schmelzpunkt |
|---|---|---|
| 105 | 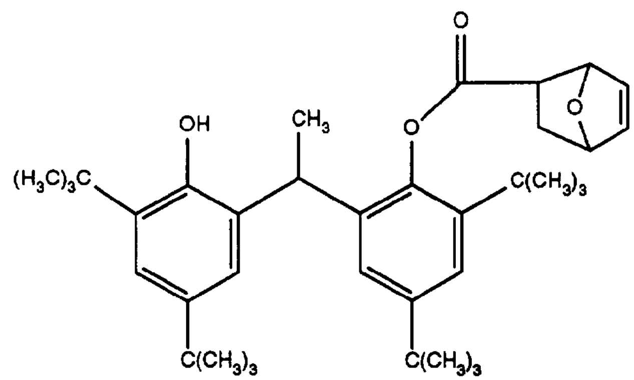 | Elementaranalyse:<br>C% H%<br>ber.: 79,24 9,35<br>gef.: 78,78 9,50<br>Schmelzpunkt: 95-96°C |
| 106 | 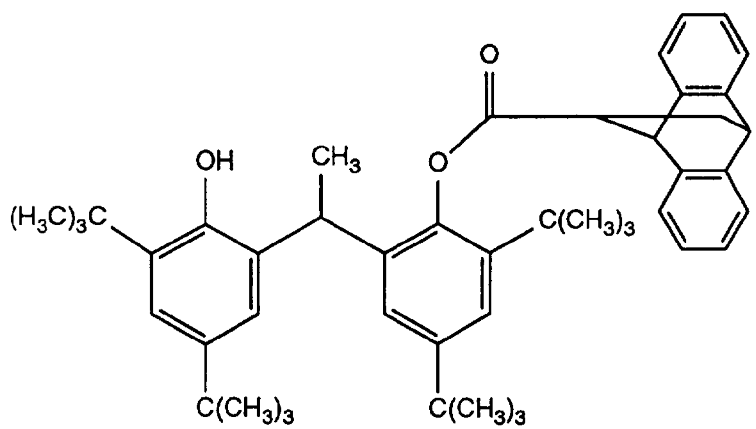 | Elementaranalyse:<br>C% H%<br>ber.: 84,13 8,71<br>gef.: 83,94 8,85<br>Schmelzpunkt: 217-219°C |

Die Verbindungen der Formel I eignen sich zum Stabilisieren von organischen Materialien gegen thermischen,

oxidativen oder lichtinduzierten Abbau. Beispiele für derartige Materialien sind:

1. Polymere von Mono- und Diolefinen, beispielsweise Polypropylen, Polyisobutylen, Polybuten-1, Poly-4-methylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder A-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol,

Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide, Polyetherimide, Polyesterimide, Polyhydantoine und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.

26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Ein weiterer Gegenstand der Erfindung ist daher eine Zusammensetzung enthaltend ein gegen oxidativen, thermischen oder lichtinduzierten Abbau empfindliches organisches Material und mindestens eine Verbindung der Formel I.

Bei dem organischen Material handelt es sich vorzugsweise um synthetische Polymere, insbesondere solche aus den oben angegebenen Gruppen. Polyolefine und ein in Lösung polymerisierter Polybutadien-Kautschuk sind besonders bevorzugt. Ebenfalls besonders bevorzugt ist ein in Lösung polymerisiertes Styrol-Butadien-Copolymer oder Styrol-Butadien-Blockcopolymer, worin das Verhältnis von Styrol zu konjugiertem Butadien zum Beispiel 5:95 bis 95:5 beträgt. Bevorzugt liegt der Anteil von Polybutadien in diesen Copolymeren bei 5 bis 30%.

Im allgemeinen werden die Verbindungen der Formel I dem zu stabilisierenden organischen Material in Mengen von 0,01 bis 10 %, vorzugsweise 0,01 bis 5 %, insbesondere 0,05 bis 0,5 %, bezogen auf das Gesamtgewicht des zu stabilisierenden Materials, zugesetzt.

Die Einarbeitung der erfindungsgemässen Verbindungen in das organische Material kann nach bekannten Methoden erfolgen, beispielsweise vor oder während der Formgebung oder durch Aufbringen der gelösten oder dispergierten Verbindungen auf das organische Material, gegebenenfalls unter nachträglichem Verdunsten des Lösungsmittels. Die erfindungsgemässen Verbindungen können als Pulver, Granulat oder auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden, z.B. auch während einer Lösungspolymerisation vor Abdampfen des Lösungsmittels.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die stabilisierten organischen Materialien der Erfindung können zusätzlich auch verschiedene herkömmliche Additive enthalten, wie beispielsweise:

1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tertbutyl-4-hydroxyanisol, 3,5-Di-tert-butyl-4-hydroxyphenyl-stearat, Bis(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis(6-tert-butyl-2-methylphenol), 4,4'-Thio-bis(3,6-di-sec.-amylphenol), 4,4'-Bis(2,6-dimethyl-4-hydroxyphenyl)-disulfid.

1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis-(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methylbenzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tertbutyl-4-hydroxy-2-methylphenyl)-4-n-dodecylmercapto-butan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.

1.9. Hydroxybenzyl-Aromaten, z.B. 1,3,5-Tris(3,5-di-tert-butyl-4-hydroxybenzyl)-2,4,6-trimethylbenzol, 1,4-Bis (3,5-di-tert-butyl-4-hydroxybenzyl)-2,3,5,6-tetramethylbenzol, 2,4,6-Tris(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)- 1,3,5-triazin, 2-Octylmercapto-4,6-bis(3,5-di-tert-butyl-4-hydroxyanilino)- 1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-iso-cyanurat, 2,4,6-Tris(3,5-di-tert-butyl-4-hydroxyphenylethyl)- 1,3,5-triazin, 1,3,5-Tris(3,5-di-tert-butyl-4-hydroxyphenylpropionyl)-hexahydro-1,3,5-triazin, 1,3,5-Tris(3,5-dicyclo-hexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z. B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-iso-

cyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicydohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N, N'-Bis(3,5-di-tert-butyl-4-hydroxyphenylpropionyl)-hydrazin.

1.18. Ascorbinsäure (Vitamin C).

1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis (1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Di-methyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluolsulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tertoctyldiphenylamin, 4-n-Butylaminophenol, 4-Butyrylamino-phenol, 4-Nonanoylaminophenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N, N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tertamyl-2'-hydroxyphenyl)-benzotriazol, 2-(3',5'-Bis(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)_3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tertbutylphenylester, 3,5-Di-tert-butyl-4-hydroxybenzoesäurehexadecylester, 3,5-Di-tertbutyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl- 1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor- 1,3,5-triazin, Kondensationsprodukt aus 2-Chlor-4,6-di-(4-n-butylamino-2,2,6,6-tetramethylpiperidyl)- 1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)pyrrolidin-2,5-dion, Gemisch von 4-Hexadecyloxy- und 4-Stearyloxy-2,2,6,6-tetramethylpiperidin, Kondenstionsprodukt aus N,N'-Bis (2,2,6,6-tetramethyl-4-piperidyl)-hexamethylendiamin und 4-Cyclohexylamino-2,6-dichlor- 1,3,5-triazin, Kondensationsprodukt aus 1,2-Bis(3-aminopropylamino)-ethan und 2,4,6-trichlor- 1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro [4,5]decan, Umsetzungsprodukt von 7,7,9,9-Tetramethyl-2-cycloundecyl-1-oxa-3,8-diaza-4-oxospiro[4,5]decan und Epichlorhydrin.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tertbutyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)4'6-bis(2,4-dimethylphenyl)- 1,3,5-triazin, 2-(2,4-Dihydroxyphenyl)-4,6-bis(2,4-dimethylphenyl)-1,3,5-triazin, 2,4-Bis(2-hydroxy4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy) phenyl]4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)- 1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin.

3. Metalldesaktivatoren, wie z.B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis (benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris (nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tertbutyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz-[d,g]-1,3,2-dioxaphosphocin, Bis(2,4-di-tert-butyl-6-methylphenyl)-methylphosphit, Bis(2,4-di-tert-butyl-6-methylphenyl)-ethylphosphit.

5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Diakylhydroxylamin aus hydrierten Talgfettaminen.

6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecylnitron, N-Hexadecyl-alpha-pentadecyl-nitron, N-Octadecyl-alpha-heptadecyl-nitron, N-Hexadecyl-alpha-heptadecyl-nitron, N-Ocatadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.

7. Thiosynergisten wie z.B. Thiodipropionsäure-di-laurylester oder Thiodipropionsäure-di-stearylester.

8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyldithiocarbamat, Dioctadecyldisulfid, Pentaerythrit-tetrakis(β-dodecylmercapto)-propionat.

9. Polyamidstabilisatoren, wie z.B. Kupfersalze in Kombination mit Jodiden und/oder Phosphorverbindungen und Salze des zweiwertigen Mangans.

10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").

12. Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.

13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.

14. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Das Gewichtsverhältnis von erfindungsgemässen Verbindungen zu den herkömmlichen Additiven kann beispielsweise 1:0,5 bis 1:5 betragen.

Ein weiterer Gegenstand der Erfindung ist die Verwendung der Verbindungen der Formel I zum Stabilisieren von organischem Material gegen oxidativen, thermischen oder lichtinduzierten Abbau.

Die folgenden Beispiele erläutern die Erfindung weiter. Alle Mengenangaben entsprechen Gewichtsteilen sofern nichts anderes angegeben ist.

Beispiel 1: Herstellung der Verbindung 101.

Ein Gemisch von 1,5 g (3 mMol) 2,2'-Ethyliden-bis[4,6-di-tert-butylphenol]monoacrylat (Herstellung z.B. in Analogie zu EP-A-500 323) und 1,4 g (21 mMol) Cyclopentadien in 40 ml Toluol wird auf 90°C erwärmt und während zwei Stunden bei dieser Temperatur gerührt. Anschliessend wird das Reaktionsgemisch am Vakuumrotationsverdampfer eingeengt. Die Kristallisation des erhaltenen Rohproduktes aus Acetonitril liefert 1,5 g (91% der Theorie) der Verbindung 101 als weisses Pulver (Isomerengemisch).

Schmelzpunkt: 150-154°C

| Elementaranalyse: | C% | H% |
|---|---|---|
| berechnet: | 81,67 | 9,74 |
| gefunden: | 81,86 | 9,68 |

Beispiel 2: Herstellung der Verbindung 101.

Ein Gemisch von 4,93 g (10 mMol) 2,2'-Ethyliden-bis[4,6-di-tert-butylphenol]monoacrylat und 4,6 g (70 mMol) Cyclopentadien in 20 ml Isopropanol und 20 ml Wasser wird auf 90°C erwärmt und während zwei Stunden bei dieser Temperatur gerührt. Anschliessend wird das Reaktionsgemisch am Vakuumrotationsverdampfer eingeengt, der Rückstand auf Wasser gegossen und mit Essigester zweimal extrahiert Die organischen Phasen werden vereinigt, über Natriumsulfat getrocknet und am Vakuumrotationsverdampfer eingedampft. Die Kristallisation des Rückstandes aus Acetonitril liefert 3,9 g (71% der Theorie) der Verbindung 101 als weisses Pulver (Isomerengemisch).

Schmelzpunkt: 148-150°C

| Elementaranalyse: | C% | H% |
|---|---|---|
| berechnet: | 81,67 | 9,74 |
| gefunden: | 81,69 | 9,68 |

Beispiel 3: Herstellung der Verbindung 101.

In einem 100 ml Rundkolben werden unter $N_2$-Atmosphäre 9,85 g (20 mMol) 2,2'-Ethyliden-bis[4,6-di-tert-butylphenol]monoacrylat, 0,6 ml (1,08 mMol = 5 Mol%) einer 1,8 M Ethylaluminiumdichloridlösung in Toluol und 50 ml Toluol vorgelegt. Zu dieser Suspension wird bei Raumtemperatur 1,8 ml (22 mMol) Cyclopentadien getropft und das Gemisch während 2 1/2 Stunden gerührt Anschliessend wird die erhaltene orange Lösung auf 50 ml HCl (1 M) gegossen und zweimal mit Essigester extrahiert. Die organischen Phasen werden vereinigt, mit einer wässrigen, gesättigten NaCl-Lösung gewaschen, über Natriumsulfat getrocknet und am Vakuumrotationsverdampfer eingeengt Die Kristallisation des Rückstandes (10,5 g) aus Acetonitril liefert 8,5 g (77% der Theorie) der Verbindung 101 als weisses Pulver (Isomerengemisch).

Schmelzpunkt: 146-150°C

| Elementaranalyse: | C% | H% |
|---|---|---|
| berechnet: | 81,67 | 9,74 |
| gefunden: | 81,70 | 9,87 |

Beispiel 4: Herstellung der Verbindung 101.

In einem 0,3 l Autoklaven werden 49,3 g (0,1 Mol) 2,2'-Ethyliden-bis[4,6-di-tert-butylphenol]monoacrylat, 7,9 g (0,06 Mol) Dicyclopentadien und 50 ml Toluol vorgelegt Der Autoklav wird inertisiert ($N_2$) und während 8 Stunden auf 200°C erwärmt (Maximaldruck 3 bar). Anschliessend wird die auf Raumtemperatur abgekühlte erhaltene gelbe Lösung am Vakuumrotationsverdampfer eingeengt. Die Kristallisation des Rückstandes aus Acetonitril liefert 44,5 g (80% der Theorie) der Verbindung 101 als weisses Pulver (Isomerengemisch).

Schmelzpunkt: 157-160°C

| Elementaranalyse: | C% | H% |
|---|---|---|
| berechnet: | 81,67 | 9,74 |
| gefunden: | 81,81 | 9,85 |

Beispiel 5: Herstellung der Verbindung 101.

Ein Gemisch von 4,4 g (10 mMol) 2,2'-Ethyliden-bis[4,6-di-tert-butylphenol] und 2 g (13 mMol) Norbon-5-en-2-carbonylchlorid (Herstellung z.B. in Analogie zu J. Org. Chem. 38, 642 (1973)) wird auf 140°C erwärmt und während 5 Minuten bei dieser Temperatur gerührt, wobei ab 125°C eine HCl-Entwicklung entsteht. Nach dem Abkühlen auf ca.

110°C werden 15 ml Acetonitril zugegeben und das Gemisch auf Raumtemperatur gekühlt. Das auskristallisierte Produkt wird abfiltriert und am Hochvakuum getrocknet. Man erhält 4,2 g (70% der Theorie) der Verbindung 101 als weisses Pulver (Isomerengemisch).

Schmelzpunkt: 152-155°C

| Elementaranalyse: | C% | H% |
|---|---|---|
| berechnet: | 81,67 | 9,74 |
| gefunden: | 81,65 | 9,96 |

Die Verbindungen 102 und 103 werden aus Methylen-bis[4-methyl-6-tert-butylphenol]monoacrylat bzw. 2,2'-Ethyliden-bis[4,6-di-tert-pentylphenol]monoacrylat und Cyclopentadien nach der im Beispiel 3 beschriebenen Methode hergestellt.

Die Verbindung 102 bis wird in Analogie zu dem im Beispiel 4 beschriebenen Verfahren aus Methylcyclopentadien-Dimer und 2,2'-Ethyliden-bis[4,6-di-tert-butylphenol] monoacrylat hergestellt

Beispiel 6: Herstellung der Verbindung 104.

Zu einer auf 5-10°C gekühlten Lösung von 12,7 g (29 mMol) 2,2'-Ethyliden-bis[4,6-di-tert-butylphenol] in 50 ml Tetrahydrofuran wird langsam unter $N_2$-Atmosphäre 18 ml (29 mMol) einer 1,6 N Butyllithium-Hexan-Lösung getropft. Das Gemisch wird während 15 Minuten gerührt und anschliessend werden 6,5 g (38 mMol) 2-Methyl-norbon-5-encarbonylchlorid (Herstellung z.B. in Analogie zu A. F. Jacobine et al.; J. of Appl. Polym. Sci. 45, 471 (1992)) in 6 ml Tetrahydrofuran zugetropft. Das Gemisch wird über Nacht am Rückfluss gekocht. Nach dem Abkühlen auf Raumtemperatur wird das Reaktionsgemisch über Hyflo filtriert und am Vakuumrotationsverdampfer eingedampft. Die Kristallisation des Rückstandes aus Acetonitril liefert 11,7 g (70% der Theorie) der Verbindung 104 als weisses Pulver (Isomerengemisch).

Schmelzpunkt: 132-140°C

Beispiel 7: Herstellung der Verbindung 105.

In einem 500 ml Rundkolben werden unter $N_2$-Atmosphäre 39,5 g (0,09 Mol) 2,2'-Ethyliden-bis[4,6-di-tert-butylphenol] und 11,84 g (0,12 mMol) Triethylamin in 200 ml Toluol vorgelegt. Zu dieser farblosen Lösung wird bei -20°C 16,5 g (0,1 Mol) 7-Oxabicyclo[2,2,1]hept-5-en-2-carbonylchlorid (Herstellung z.B. in Analogie zu Bull. Chem. Soc. Jpn., 57, 3339-40 (1984)) zugetropft. Das Gemisch wird während 30 Minuten bei -20°C gerührt und dann innerhalb von 2 Stunden auf Raumtemperatur gebracht. Die Salze werden abfiltriert und das Filtrat am Vakuumrotationsverdampfer eingeengt. Die Chromatographie des Rückstandes auf Silicagel mit dem Laufmittel Hexan-Essigester 80:1 liefert 11,2 g (22% der Theorie) der Verbindung 105 als weisses Pulver (Isomerengemisch).

Schmelzpunkt: 95-96°C

Beispiel 8: Herstellung der Verbindung 106.

In einem 250 ml Rundkolben werden unter $N_2$-Atmosphäre 6,6 g (15 mMol) 2,2'-Ethyliden-bis[4,6-di-tert-butylphenol] und 4,2 g (15,7 mMol) 9,10-Dihydro-9-10-ethanoantracen-11-carbonylchlorid (Herstellung z.B. in Analogie zu J. A.C.S. 94, 1193 (1972)) in 100 ml Toluol vorgelegt. Zu dieser Lösung wird bei ca. 5°C 2,7 ml (19,5 mMol) Triethylamin getropft Das Gemisch wird während zwei Stunden bei 5°C gerührt, danach auf Wasser gegossen und zweimal mit Essigester extrahiert. Die organischen Phasen werden vereinigt, mit einer wässrigen, gesättigten Natriumchloridlösung gewaschen, über Natriumsulfat getrocknet und am Vakuumrotationsverdampfer eingeengt. Die Kristallisation des Rückstandes aus Methanol liefert 6,6 g (62 % der Theorie) der Verbindung 106 als hellgelbes Pulver (Isomerengemisch).

Schmelzpunkt: 217-219°C

Beispiel 9: Stabilisierung von Polypropylen bei Mehrfachextrusion.

1,3 kg Polypropylenpulver (®Profax 6501), das mit 0,025 % 3-[3,5-di-tert-butyl-4-hydroxyphenyl]propionsäure-n-octadecylester vorstabilisiert wurde und bei 230°C und 2,16 kg einen Schmelzindex von 3,2 besitzt, werden mit 0,05 % Pentaerythrityl-tetrakis-[3-(3,5-di-tert-butyl-4-hydroxyphenyl)-propionat]), 0,05 % Calciumstearat, 0,03 % Dihydrotalcit [$Mg_{4,5}Al_2(OH)_{13}CO_3 \cdot 3,5\ H_2O$] und 0,05 % einer Verbindung aus Tabelle 1 gemischt. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert. Dieses Granulat wird wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 2 zusammengefasst.

Tabelle 2:

| Verbindung aus Tabelle 1 | Schmelzindex nach 3 Extrusionen |
|---|---|
| - | 20,0 |
| 101 | 6,8 |
| 104 | 7,2 |

Beispiel 10: Stabilisierung von Elastomeren (Brabender-Test).

Ein Styrol-Butadien-Styrol Elastomer (®Finaprene 902) wird mit dem in Tabelle 3 angegebenen Stabilisator versetzt und in einem Brabender Plastographen bei 200°C mit 60 Umdrehungen/Minute geknetet. Der Knetwiderstand wird als Drehmoment kontinuierlich registriert. Infolge der Vernetzung des Polymeren erfolgt im Laufe der Knetzeit nach anfänglicher Konstanz eine rasche Zunahme des Drehmoments. Die Wirksamkeit der Stabilisatoren äussert sich in einer Verlängerung der Zeitspanne, in der das Drehmoment konstant bleibt. Die erhaltenen Werte sind in Tabelle 3 aufgeführt.

Tabelle 3:

| 0,25% Stabilisator aus Tabelle 1 | Zeit in Minuten bis zum Drehmomentanstieg |
|---|---|
| - | 24,2 |
| 101 | 53 |
| 104 | 62 |

Beispiel 11: Stabilisierung von Styrol-Butadienblock-Polymerisaten.

Bei thermisch oxidativer Schädigung von Styrol-Butadienblock-Polymeren erfolgt eine Vernetzung der Kautschukphase. Diese Vernetzung führt während der Verarbeitung in einem Extruder oder in einer Spritzgussmaschine zu einer Steigerung der Schmelzviskosität und daher des Extrusionsdrucks.

Die Prüfung der Verarbeitungsstabilität von Styrol-Butadienblock-Polymeren wird häufig in einem Kapillarrheometer durchgeführt. Dabei wird das Polymerisat, ähnlich wie bei der Extrusion, als Schmelze durch eine Düse gedrückt.

25 g Granulat von Styrol-Butadienblock-Polymerisat (®K-Resin KR-01 von ®Phillips Petroleum) enthaltend 0,2% n-Octadecyl-3-[3',5'-di-tert-butyl-4'-hydroxyphenyl]propionat und 0,6% Tris[nonylphenyl]phosphit werden in 250 ml Cyclohexan bei Raumtemperatur gelöst.

Die in Tabelle 4 angegebene Menge an Stabilisator wird in Toluol gelöst und der Polymerlösung beigemischt. Anschliessend wird das Cyclohexan bei 60°C und 0,013 bar abgedampft.

Das erhaltene Polymer wird bei 180°C zu 2 mm dicken Platten verpresst, aus denen kreisförmige Proben mit 8 mm Durchmesser gestanzt werden. Diese Proben werden in den Vorlagekanal eines Kapillarrheometers des Typs ®Keyeness Galaxy V eingefüllt und bei 250°C mit einer Schergeschwindigkeit von 14,594 $sec^{-1}$ vermessen. Nach einer Verlaufzeit von 6 Minuten wird die scheinbare Schergeschwindigkeit während 30 Minuten als Funktion der Zeit registriert. Die Steigung dieser Kurve ($\Delta\eta/\Delta t$ in [Pa sec/min]) ist ein Mass für den Vernetzungsgrad des Polymers und steht daher in direktem Zusammenhang mit der Wirkung des Stabilisators. Je kleiner dieser Wert ist, desto wirksamer ist der Stabilisator.

Die Ergebnisse sind in Tabelle 4 aufgeführt.

Tabelle 4:

| 0,15% Stabilisator aus Tabelle 1 | $\frac{\Delta\eta \text{ (Pa sec)}}{\Delta t \text{ (min)}}$ |
|---|---|
| - | 23 |
| 101 | 5 |
| 102 | 7 |
| 103 | 4 |
| 104 | 2 |
| 105 | 4 |

## Patentansprüche

**1.** Verbindung der Formel I,

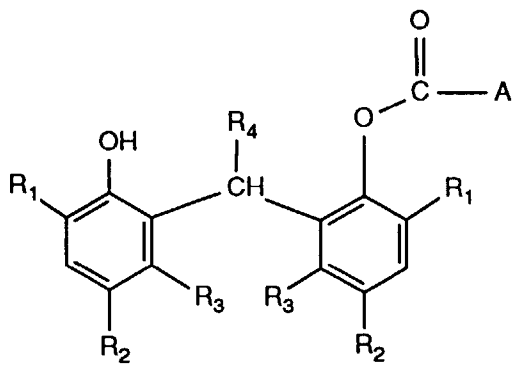

(I)

worin A eine Gruppe der Formel IIa oder IIb bedeutet,

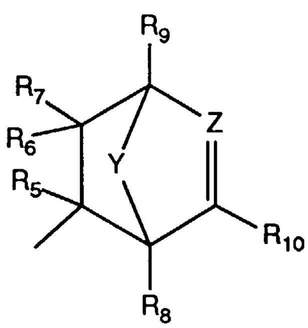

(IIa),

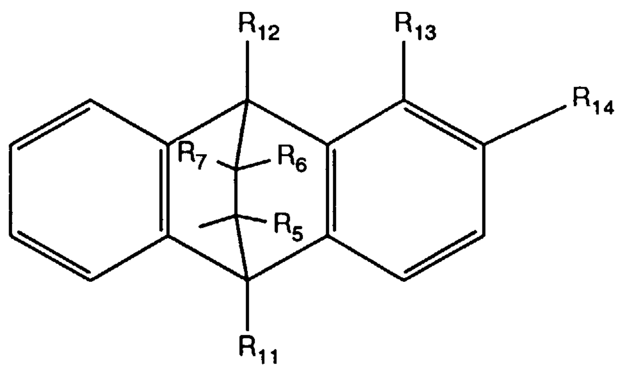

(IIb)

Y Sauerstoff, Methylen, Ethyliden oder eine Gruppe >C=C($CH_3$)$_2$ ist,

Z Stickstoff,

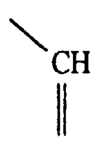

oder

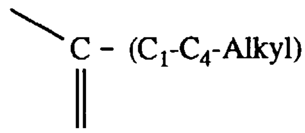

darstellt,

die Reste $R_1$ unabhängig voneinander $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl oder -$CH_2$-S-$X_1$ bedeuten,

die Reste $R_2$ unabhängig voneinander Wasserstoff, $C_1$-$C_{25}$-Alkyl, $C_2$-$C_{24}$-Alkenyl, $C_5$-$C_{112}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-Alkyl substituiertes Phenyl, $C_7$-$C_9$-Phenylalkyl, -$CH_2$-S-$X_1$, -$(CH_2)_p$COO-$X_2$ oder -$(CH_2)_q$O-$X_3$ sind,

die Reste $R_3$ unabhängig voneinander Wasserstoff oder $C_1$-$C_4$-Alkyl darstellen,

$R_4$ Wasserstoff oder $C_1$-$C_8$-Alkyl ist,

$R_5$ Wasserstoff, $C_1$-$C_{10}$-Alkyl, Phenyl, -$CH_2$-COO-$X_4$ oder CN bedeutet,

$R_6$ Wasserstoff, $C_1$-$C_4$-Alkyl, Phenyl, -COO-$X_5$, -CN oder -CON($X_6$)($X_7$) ist,

$R_7$ Wasserstoff oder $C_1$-$C_{10}$-Alkyl bedeutet,

$R_8$, $R_9$ und $R_{10}$ unabhängig voneinander Wasserstoff oder $C_1$-$C_4$-Alkyl sind,

$R_{11}$ und $R_{12}$ unabhängig voneinander Wasserstoff, $C_1$-$C_4$-alkyl oder Phenyl bedeuten,

$R_{13}$ und $R_{14}$ unabhängig voneinander Wasserstoff oder $C_1$-$C_4$-Alkyl sind,

$X_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl oder -$(CH_2)_r$COO-$Y_1$ darstellt,

$X_2$, $X_4$ und $X_5$ unabhängig voneinander $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl bedeuten,

$X_3$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl, $C_7$-$C_9$-Phenylalkyl, $C_1$-$C_{25}$-Alkanoyl, $C_3$-$C_{25}$-Alkenoyl, durch Sauerstoff, Schwefel oder >N-$Y_2$ unterbrochenes $C_3$-$C_{25}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$X_6$ und $X_7$ unabhängig voneinander Wasserstoff, $C_1$-$C_{25}$-Alkyl, $C_2$-$C_{24}$-Alkenyl, $C_5$-$C_{12}$-Cycloalkyl, durch $C_1$-$C_4$-Alkyl substituiertes $C_5$-$C_{12}$-Cycloalkyl, $C_5$-$C_{12}$-Cycloalkenyl, durch $C_1$-$C_4$-Alkyl substituiertes $C_5$-$C_{12}$-Cycloalkenyl, Phenyl, durch $C_1$-$C_4$-FSB substituiertes Phenyl oder $C_7$-$C_9$-Phenylalkyl sind,

$Y_1$ $C_1$-$C_{25}$-Alkyl, $C_5$-$C_{12}$-Cycloalkyl, durch $C_1$-$C_4$-Alkyl substituiertes $C_5$-$C_{12}$-Cycloalkyl, Phenyl, durch $C_1$-$C_4$-Alkyl substituiertes Phenyl oder $C_7$-$C_9$-Phenylalkyl ist,

$Y_2$ Wasserstoff oder $C_1$-$C_8$-Alkyl bedeutet,

p 0, 1 oder 2 ist,

q eine ganze Zahl von 0 bis 8 darstellt,

r 1 oder 2 bedeutet.

**2.** Verbindung gemäss Anspruch 1, worin

die Reste $R_1$ unabhängig voneinander $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl, $C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl oder $-CH_2$-S-$X_1$ bedeuten,

die Reste $R_2$ unabhängig voneinander Wasserstoff, $C_1$-$C_{18}$-Alkyl, $C_2$-$C_{18}$-Alkenyl, $C_5$-$C_8$-Cycloalkyl, durch $C_1$-$C_4$-Alkyl substituiertes $C_5$-$C_8$-Cycloalkyl,
$C_5$-$C_8$-Cycloalkenyl, durch $C_1$-$C_4$-Alkyl substituiertes $C_5$-$C_8$-Cycloalkenyl, Phenyl, durch $C_1$-$C_4$-Alkyl substituiertes Phenyl, $C_7$-$C_9$-Phenylalkyl, $-CH_2$-S-$X_1$, $-(CH_2)_p$COO-$X_2$ oder $-(CH_2)_q$O-$X_3$ sind,

$R_5$ Wasserstoff, $C_1$-$C_{10}$-Alkyl, Phenyl oder CN bedeutet,

$R_6$ Wasserstoff, $C_1$-$C_4$-Alkyl oder Phenyl ist,

$X_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl oder $-(CH_2)_r$COO-$Y_1$ darstellt,

$X_2$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl bedeutet,

$X_3$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl, $C_7$-$C_9$-Phenylalkyl, $C_1$-$C_{18}$-Alkanoyl, $C_3$-$C_{18}$-Alkenoyl, durch Sauerstoff oder >N-$Y_2$ unterbrochenes $C_3$-$C_{18}$-Alkanoyl, $C_6$-$C_9$-Cycloalkylcarbonyl, Benzoyl, durch $C_1$-$C_4$-Alkyl substituiertes Benzoyl, Thenoyl oder Furoyl ist,

$Y_1$ $C_1$-$C_{18}$-Alkyl, $C_5$-$C_8$-Cycloalkyl, durch $C_1$-$C_4$-Alkyl substituiertes $C_5$-$C_8$-Cycloalkyl, Phenyl, durch $C_1$-$C_4$-Alkyl substituiertes Phenyl oder $C_7$-$C_9$-Phenylalkyl ist.

**3.** Verbindung gemäss Anspruch 1, worin

die Reste $R_1$ unabhängig voneinander $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl oder $-CH_2$-S-$X_1$ bedeuten,

die Reste $R_2$ unabhängig voneinander Wasserstoff, $C_1$-$C_{10}$-Alkyl, $C_2$-$C_{18}$-Alkenyl, $C_5$-$C_8$-Cycloalkyl, $C_5$-$C_8$-Cycloalkenyl, Phenyl, $C_7$-$C_9$-Phenylalkyl, $-CH_2$-S-$X_1$, $-(CH_2)_p$COO-$X_2$ oder $-(CH_2)_q$O-$X_3$ sind,

die Reste $R_3$ Wasserstoff darstellen,

$R_4$ Wasserstoff oder $C_1$-$C_4$-Alkyl ist,

$R_5$ Wasserstoff, $C_1$-$C_4$-Alkyl, Phenyl oder CN bedeutet,

$R_6$ Wasserstoff, $C_1$-$C_4$-Alkyl oder Phenyl ist,

$R_7$ Wasserstoff oder $C_1$-$C_4$-Alkyl bedeutet,

$X_1$ $C_1$-$C_{10}$-Alkyl, $C_5$-$C_8$-Cycloalkyl, Phenyl, $C_7$-$C_9$-Phenylalkyl oder $-(CH_2)_r$COO-$Y_1$ darstellt,

$X_2$ $C_1$-$C_{10}$-Alkyl, $C_5$-$C_8$-Cycloalkyl, Phenyl oder $C_7$-$C_9$-Phenylalkyl bedeutet,

$X_3$ $C_1$-$C_{10}$-Alkyl, $C_5$-$C_8$-Cycloalkyl, Phenyl, $C_7$-$C_9$-Phenylalkyl, $C_1$-$C_{10}$-Alkanoyl, $C_3$-$C_{18}$-Alkenoyl, durch Sauerstoff unterbrochenes $C_3$-$C_{18}$-Alkanoyl oder Benzoyl ist,

$Y_1$ $C_1$-$C_{10}$-Alkyl, $C_5$-$C_8$-Cycloalkyl, Phenyl oder $C_7$-$C_9$-Phenylalkyl ist.

**4.** Verbindung gemäss Anspruch 1, worin $R_8$, $R_9$, $R_{10}$, $R_{11}$, $R_{12}$, $R_{13}$ und $R_{14}$ Wasserstoff bedeuten.

**5.** Verbindung gemäss Anspruch 1, worin

die Reste $R_1$ unabhängig voneinander $C_1$-$C_5$-Alkyl oder $C_5$-$C_8$-Cycloalkyl bedeuten,

die Reste $R_2$ unabhängig voneinander $C_1$-$C_5$-Alkyl darstellen,

die Reste $R_3$ Wasserstoff bedeuten und

$R_4$ Wasserstoff oder $C_1$-$C_4$-Alkyl ist.

**6.** Verbindung gemäss Anspruch 1, worin

die Reste $R_1$ gleich sind und verzweigtes $C_3$-$C_5$-Alkyl bedeuten,

die Reste $R_2$ gleich sind und $C_1$-$C_5$-Alkyl darstellen,

die Reste $R_3$ Wasserstoff bedeuten und

$R_4$ Wasserstoff oder $C_1$-$C_4$-Alkyl ist.

**7.** Verbindung gemäss Anspruch 1, worin

Y Sauerstoff oder Methylen bedeutet,

Z eine Gruppe

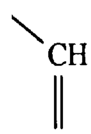

ist,

die Reste $R_1$ gleich sind und verzweigtes $C_3$-$C_5$-Alkyl bedeuten,

die Reste $R_2$ gleich sind und $C_1$-$C_5$-Alkyl darstellen,

die Reste $R_3$ Wasserstoff bedeuten,

$R_4$ Wasserstoff oder $C_1$-$C_4$-Alkyl ist,

$R_5$ Wasserstoff, $C_1$-$C_4$-Alkyl oder CN darstellt,

$R_6$, $R_7$, $R_8$, $R_9$, $R_{10}$, $R_{11}$, $R_{12}$, $R_{13}$ und $R_{14}$ Wasserstoff sind.

**8.** Die Verbindung

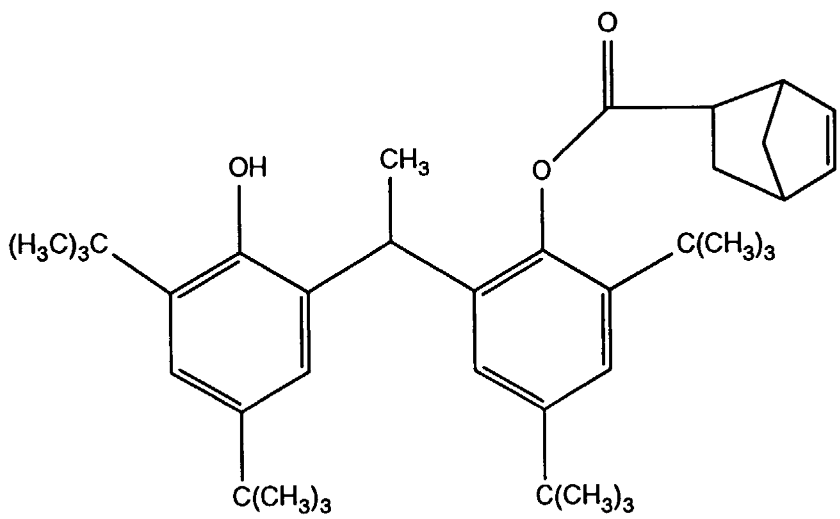

gemäss Anspruch 1.

**9.** Zusammensetzung enthaltend ein gegen oxidativen, thermischen oder lichtinduzierten Abbau empfindliches organisches Material und mindestens eine Verbindung der Formel I gemäss Anspruch 1.

**10.** Zusammensetzung gemäss Anspruch 9, worin das organische Material ein synthetisches Polymer ist.

**11.** Zusammensetzung gemäss Anspruch 9, worin das organische Material ein Polyolefin ist.

**12.** Zusammensetzung gemäss Anspruch 9, worin das organische Material ein in Lösung polymerisierter Polybutadien-Kautschuk ist.

**13.** Zusammensetzung gemäss Anspruch 9, worin das organische Material ein in Lösung polymerisiertes Styrol-Butadien-Copolymer oder Styrol-Butadien-Blockcopolymer ist

**14.** Verwendung einer Verbindung der Formel I gemäss Anspruch 1 zum Stabilisieren eines organischen Materials gegen oxidativen, thermischen oder lichtinduzierten Abbau.

Europäisches Patentamt

# EUROPÄISCHER RECHERCHENBERICHT

Nummer der Anmeldung
EP 96 81 0063

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DE-A-29 48 969 (ASAHI KASEI KOGYO K.K.)<br>* Seite 1 - Seite 6; Ansprüche *<br>Verbindung 16<br>* Seite 13; Tabelle 1 *<br>* Seite 20, Absatz 4 - Seite 21, Absatz 1 *<br>----- | 1 | C07C69/753<br>C07D307/93<br>C08K5/134 |

RECHERCHIERTE SACHGEBIETE (Int.Cl.6)
C07C

Der vorliegende Recherchenbericht wurde für alle Patentansprüche erstellt

| Recherchenort | Abschlußdatum der Recherche | Prüfer |
|---|---|---|
| DEN HAAG | 8.Mai 1996 | Kinzinger, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)